# EUROPÄISCHE PATENTANMELDUNG

(11) **EP 0 889 030 A1**
(43) Veröffentlichungstag der Anmeldung: **07.01.1999**
(21) Anmeldenummer: 98111637.9
(22) Anmeldetag: 24.06.1998
(51) Int. Cl.: C07C 321/14, C10M 135/22

(54) **Polysulfide, ein Verfahren zu ihrer Herstellung und ihre Verwendung**

(30) Priorität: 05.07.1997 DE 19728806
(71) Anmelder: RHEIN-CHEMIE RHEINAU GmbH, 68219 Mannheim (DE)
(72) Erfinder: Fessenbecker, Achim, Dr., 76669 Bad Schönborn (DE); Hegmann, Joachim, Dr., 67117 Limburgerhof (DE); Pauli, Alfred, Dr., 68799 Reilingen (DE); Schilling, Kurt, 68723 Schwetzingen (DE); Heiliger, Ludger, Dr., 67433 Neustadt (DE)
(74) Vertreter: Steiling, Lothar, Dr.

(57) **Zusammenfassung**

Geschwefelte unverzweigte Verbindungen der Formel R₂Sₙ, worin R unverzweigte, C₄-C₂₂-Alkylreste und n 1,5 bis 3,5 bedeuten, und Verfahren zu deren Herstellung, indem endständige, unverzweigte C₄-C₂₂-Olefine, 0,5 bis 1,5 mol Schwefel und 0,4 bis 0,7 mol Schwefelwasserstoff pro Mol Olefin bei 50 bis 160°C unter 2 bis 20 bar Druck 3 bis 9 Stunden in Anwesenheit von 0,01 bis 0,5 Gew.-% (bezogen auf die Summe der Einsatzstoffe) eines anionischen Katalysators im Autoklaven umgesetzt werden.

## Beschreibung

Die Erfindung betrifft helle, geschwefelte, unverzweigte Kohlenwasserstoffe, ein Verfahren zu ihrer Herstellung und ihre Verwendung.

Geschwefelte Kohlenwasserstoffe, sogenannte 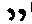 Schwefelträger" sind bekannt. Üblicherweise werden sie aus Olefinen, H₂S oder Mercaptanen und Schwefel mit Hilfe von Katalysatoren bei 50 bis 300°C in geschlossenen Apparaturen hergestellt. Die Reaktion verläuft glatt mit verzweigten Olefinen, deren C=C-Doppelbindungsgruppierung maximal 2 Wasserstoffatome aufweist und führt zu hellgelben bis bellbraunen Produkten (Farbe 0,5 - 4 nach ASTM-D 1500). Bekannt ist auch die Umsetzung von endständigen, unverzweigten Olefinen mit Schwefel und Katalysatoren, die zu dunkelbraun bis schwarzen Produkten führt. Diese weisen einen sehr hohen Anteil (> 75 %) an 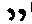 Aktiv-Schwefel" (gemessen nach Standardmethode ASTM-D 1662) auf. Im allgemeinen haben helle Schwefelträger gegenüber den dunklen den Vorteil, daß die helle Farbe des Grundöls durch die Zudosierung, selbst in hohen Konzentrationen an Additiv, erhalten bleibt. Ein Metallbearbeitungsöl z.B. bleibt somit hell und vor allem durchsichtig und erlaubt dem Anwender die visuelle Beobachtung der schmierstoffüberfluteten Schneidzone. Schwefelträger, wie z.B. das Bis-(trimethylpentyl)-pentasulfid der Formel i-(C₈H₁₇)₂-S₅, das aus Diisobutylen hergestellt wird, sind hervorragende EP-Additive in Mlneralölanwendungen, weil der Schwefel unter Druck und/oder Wärmebeanspruchung leicht in (re)aktiver Form abgespalten wird und auf Metalloberflächen durch Metallsulfidbildung einen tribologischen Effekt ausübt.

Die bekannten Schwefelträger enthalten üblicherweise 20 bis 40 Gew.-% Schwefel, Bis-(trimethylpentyl)-pentasultid beispielsweise 40 Gew-%. Die leichte Abspaltbarkeit des Schwefels - charakterisiert als 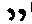 Aktiv-Schwefel" (in Anlehnung an ASTM-D 1662) - bewirkt jedoch bei Kontakt mit kupferhaltigen Materialien unerwünschte Korrosion und im Kontakt mit eisenhaltigen Oberflächen erhöhten Verschleiß. Beide Phänomene führen zur Zerstörung der Metalloberfläche. Viele Schwefelträger, auch das Bis-(trimethylpentyl)-pentasulfid, enthalten sehr viel (bis zu 95%) Schwefel in aktiver Form.

Der tribologische Effekt dieser Verbindungen kann beispielsweise durch den VKA-Test (nach DIN 51350) gemessen werden. Dabei wird der Schmierstoff (geschwefelte Kohlenwasserstoffe) durch Mischen mit einem Prüföl auf 1 Gew.-% Gesamtschwefel eingestellt, und in einem Vierkugelapparat geprüft. Der Apparat besteht aus einer rotierenden Kugel (Lautkugel), die auf drei ihr gleichen Kugeln (Standkugeln) gleitet. Die Prülkräfte (gemessen in N) können entweder stufenweise bis zum Versagen des Vierkugelapparates gesteigert werden (= > Freßlast), oder bei konstanter Prüfkraft wird nach einer festgelegten Prüfzeit der Kalottendurchmesser der drei Standkugeln ausgemessen (Verschleiß in nun). Der Schmierstoff ist um so besser, je größer die Freßlast und je kleiner der Abrieb ist (Ergebnisse siehe auch Tabelle 1).

Aufgabe der vorliegenden Erfindung war es daher, helle Schwefelträger zur Verfügung zu stellen, deren Aktivschwefelgehalt nur einen Bruchteil des Gesamtschwefelgehaltes ausmacht und die im Vergleich mit dem Stand der Technik verbesserte tribologische Eigenschaften bezüglich Freßlast und Verschleiß aufweisen und kupferhaltige Materialien weniger angreifen (korrodieren).

Diese Aufgabe konnte mit den erfindungsgemäßen Verbindungen gelöst werden.

Gegenstand der Erfindung sind Verbindungen der Formel R₂Sₙ, worin R ein unverzweigter Alkylrest mit 4 bis 22 Kohlenstoffatomen, vorzugsweise mit 8 bis 16 Kohlenstoffatomen, und n 1,5 bis 3,5, vorzugsweise 2 bis 3,5, (statistisches Mittel) ist. In diesen Verbindungen liegen ≤55% des Gesamtschwefels in Form von Aktivschwefel vor. Die Verbindungen zeigen im ob. VKA-Test bei hohen Freßlasten geringeren Verschleiß und weitaus bessere Antioxidanswirkung als die dem Stand der Technik entsprechenden verzweigten Produkte.

Die erfindungsgemäßen Verbindungen der Formel R₂Sₙ können durch Schwefelung von linearen, endständigen Olefinen mit 4 bis 22 Kohlenstoffatomen mit einem Gemisch aus Schwefel und Schwefelwasserstoff in Gegenwart von Katalysatoren in an sich bekannter Weise hergestellt werden. Dazu werden lineare endständige Olefine mit 4 bis 22 Kohlenwasserstoffatomen (also lineare 1-Olefine) mit einem Gemisch aus 0,5 bis 1,5 mol Schwefel und 0,4 - 0,7 mol Schwefelwasserstoff pro mol Olefin bei 50 bis 160°C, unter 2 bis 20 bar Druck 3 bis 9 Stunden im Autoklaven in Anwesenheit von 0,01 bis 0,5 Gew.-% eines anionischen Katalysators (bezogen auf die Summe aller Einsatzstoffe) umgesetzt.

Die erfindungsgemäßen Verbindungen werden als Additive in Mineralölen, insbesondere als Schmierstoffe eingesetzt. Sie finden Verwendung als sogenannte 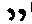 Extreme Pressure" (EP)-Zusätze (Additive) in Grundölen, aus denen vielfältige Schmierstoffendprodukte hergestellt werden, wie z.B. Metallbearbeitungsöle, Getriebeöle, Schmierfette, Hydrauliköle, Motorenöle etc..

Weiterhin erweisen sich die erfindungsgemäßen Verbindungen als sehr gute Antioxidantien. Durch ihren Einsatz in Schmierstoffen lassen sich ernorme Standzeit- bzw. Lebensdauerverlängerungen im Vergleich zu den aus dem Stand der Technik bekannten Schwefelträgern mit hohem Aktivschwefelgehalt erreichen. D.h., die die erfindungsgemäßen Verbindungen enthaltenden Schmierstoffe weisen eine wesentlich erhöhte Resistenz gegen die Zerstörung durch Sauerstoff (Oxidation) auf Dies kann anhand von genormten Schmierstoffalterungstests, z.B. in der sogenannten Rotationsbombe nach ASTM-D 2272, nachgewiesen werden (siehe auch Tabelle 2). Die oxidationsinhibierende Wirkung der erfindungsgemäßen Verbindungen zeigt sich in unterschiedlichen Grundölen. Besonders ausgeprägt ist die Antioxidanswirkung in modernen, nach dem Hydrocrack-Verfahren hergestellten Grundölen. Die erfindungsgemäßen Verbindungen weisen außerdem einen ausgeprägten Synergismus mit primären Antioxidantien, wie z.B. Diphenylaminderivaten, auf

Die Erfindung soll anhand der nachfolgenden Beispiele näher erläutert werden.

### Beispiel 1 Bis-octyl-trisulfid

- Einsatzstoffe:: 448,0 g Octen-1 = 4,0 mol
128,0 g Schwefel = 4,0 mol
0,58 g Dodecylamin = 0,1 Gew.-% auf gesamte Einwaage
72,0 g H₂S wurden eingeleitet = 2,1 mol
- Ausführung:: Der Ansatz wurde bei 20°C im Kessel dreimal mit Stickstoff gespült, 2 bar H₂S aufgedrückt und unter Rühren auf 130°C erwärmt. Bei 4,3 bar H₂S und 130°C waren nach ca. 4 Stunden 72,0 g H₂S eingeleitet.
Dann wurde die H₂S-Zufuhr abgestellt und weitere 2 Stunden gerührt, wobei der Druck auf 3,5 bar fiel. Es wurde auf 100°C abgekühlt und 2 Stunden drucklos nachgerührt.
Rohausbeute: 638,0 g gelbes Produkt. Dieses wurde 2 Stunden bei 80°C im Vakuum (50 mbar) destilliert. Ausbeute: 636,0 g
- Produktdaten:: Schwefelgehalt: 29,6 Gew.-%
Aktivschwefelgehalt: 15,4 Gew.-%
Summenformel: C₁₆H₃₄S₃ (mit R = C₈H₁₇)

### Beispiel 2

- Einsatzstoffe:: 420,0 g Decen-1 = 3,0 mol
96,0 g Schwefel = 3,0 mol
0,52 g Dodecylamin = 0,1 Gew.-% auf Gesamteinwaage
51,0g H₂S wurden eingeleitet = 1,5 mol
- Ausführung:: Wie Beispiel 1.
Rohausbeute: 563,0 g gelbes Produkt.
Dieses wurde 2 Stunden am Rotationsverdampfer bei 100°C und 15 mbar destilliert.
Destillat: 2,0 g Rückstand: 561,0 g
- Produktdaten:: Schwefelgehalt: 23,9 Gew.-%
Aktivschwefelgehalt: 12,2 Gew.-%
Summenformel: C₂₀H₄₂S_{2,8} (mit R = C₁₀H₂₁)

### Beispiel 3

- Einsatzstoffe:: 366,0 g 1-Dodecan = 2,0 mol
64,0 g Schwefel = 2,0 mol
0,4 g Dodecylamin = 0,1 Gew.-% auf gesamte Einwaage
34,0 g H₂S wurden eingeleitet = 1,0 mol
- Ausführung:: Wie in Beispiel 1.
Rohausbeute: 430,0 g gelbes Produkt.
Evtl. nicht umgesetztes Dodecen wurde wegen seines hohen Siedepunkts von 220°C eine Stunde mit Stickstoff bei 80°C ausgeblasen.
- Produktdaten:: Gesamtschwefelgehalt: 22,3 Gew.-%
Aktivschwefelgehalt: 10,5 Gew.-%
Summenformel: C₂₄H₅₀S₃ (mit R = C₁₂H₂₅)

### Beispiel 4 Bis-decyl-disulfid

- Einsatzstoffe:: 350,0 g 1-Decen = 2,5 mol
40,0 g Schwefel = 1,25 mol
0,7 g Dodecylamin = 0,1 Gew.-% auf gesamte Einwaage
45 g H₂S wurden eingeleitet = 1,3 mol
- Ausführung:: Der Ansatz wurde bei 20°C im Kessel dreimal mit Stickstoff gespült, 2 bar H₂S aufgedrückt und unter Rühren auf 130°C erwärmt. Bei 4,3 bar H₂S und 130°C waren nach 8 Stunden 45,0 g H₂S eingeleitet.
Die H₂S-Zufuhr wurde abgestellt und der Druck entspannt.
Rohausbeute: 428,0 g
Nach Destillation (2 Stunden bei 90 bis 95°C im Vakuum (ca. 20 mbar)) betrug die Ausbeute 427,0 g
- Produktdaten:: Gesamtschwefelgehalt: 17,5 Gew.-%
Aktivschwefelgehalt: 6,1 Gew.-%
Summenformel: C₂₀H₄₂S_{1,9} (mit R = C₁₀H₂₁)

### Beispiel 5

- Einsatzstoffe:: 420,0 g Decen-1 = 3,0 mol
120,0 g Schwefel = 3,75 mol
0,54 g Primene 81R = 0,1 Gew.-% auf gesamte Einwaage
54,0 g H₂S = 1,6 mol
- Ausführung:: Der Ansatz wurde im Kessel mit Stickstoff gespült, 1 bar H₂S aufgedrückt und unter Rühren auf 130°C erwärmt, wobei der H₂S-Druck auf 4,3 bar stieg. Bei 4,3 bar H₂S wurde 3 Stunden bei 130°C und 5 Stunden bei 140°C gerührt. Die H₂S-Aufnahme betrug 54,0 g. Dann wurde 2 Stunden bei 100°C mit N₂ ausgeblasen. Rohausbeute: 587,0g.
- Produktdaten:: Gesamtschwefelgehalt: 28,3 Gew.-%
Aktivschwefelgehalt: 14,5 Gew.-%
Summenformel: C₂₀H₄₂S_{3,5} (mit R = C₁₀H₂₁)

### Beispiel 6 (Vergleich mit verzweigten Produkten)

- Einsatzstoffe:: 420,0 g Diisobutylen = 3,0 mol
120,0 g Schwefel = 6 mol
0,54 g Primene 81R = 0,1 Gew.-% auf gesamte Einwaage
54,0 g H₂S = 1,6 mol
- Ausführung:: Der Ansatz wurde im Kessel mit Stickstoff gespült, 1 bar H₂S aufgedrückt und unter Rühren auf 130°C erwärmt, wobei der H₂S-Druck auf 4,3 bar stieg. Bei 4,3 bar H₂S wurde 3 Stunden bei 130°C und 5 Stunden bei 140°C gerührt. Die H₂S-Aufnahme betrug 54,0 g. Dann wurde 2 Stunden gerührt.
- Produktdaten:: Gesamtschwefelgehalt: 40 Gew.-%
Aktivschwefelgehalt: 36 Gew.-%
Summenformel: C₁₆H₃₄S₅ (mit R = C₈H₁₇)

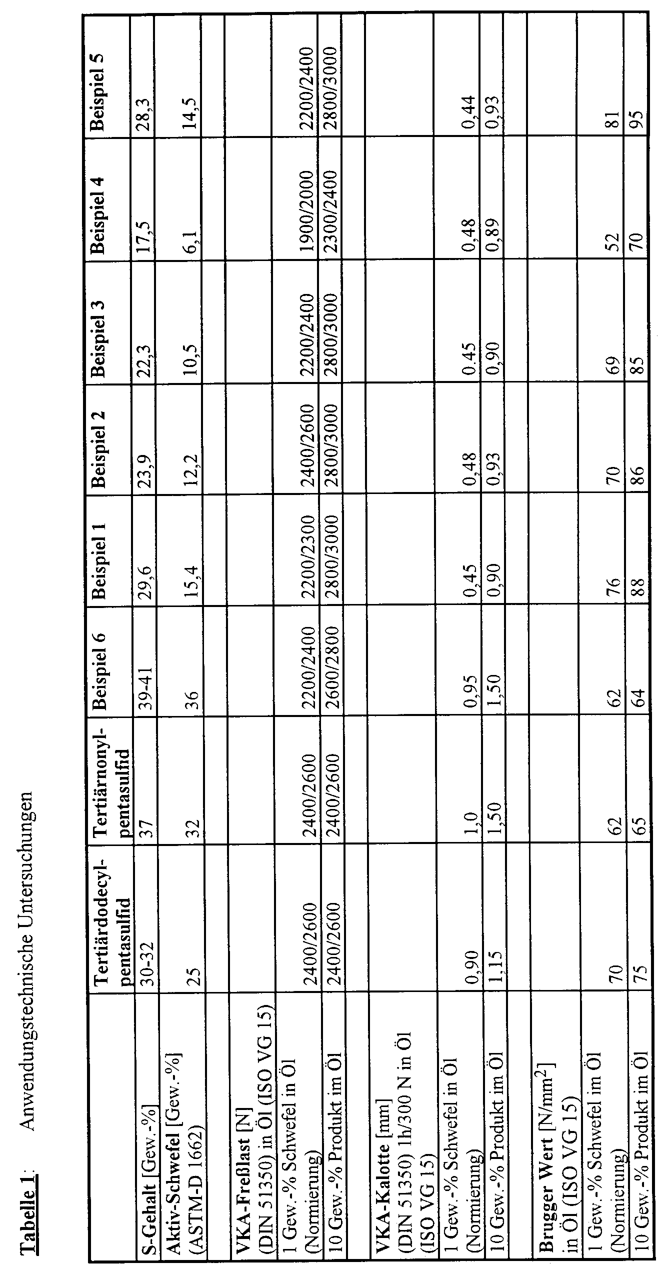

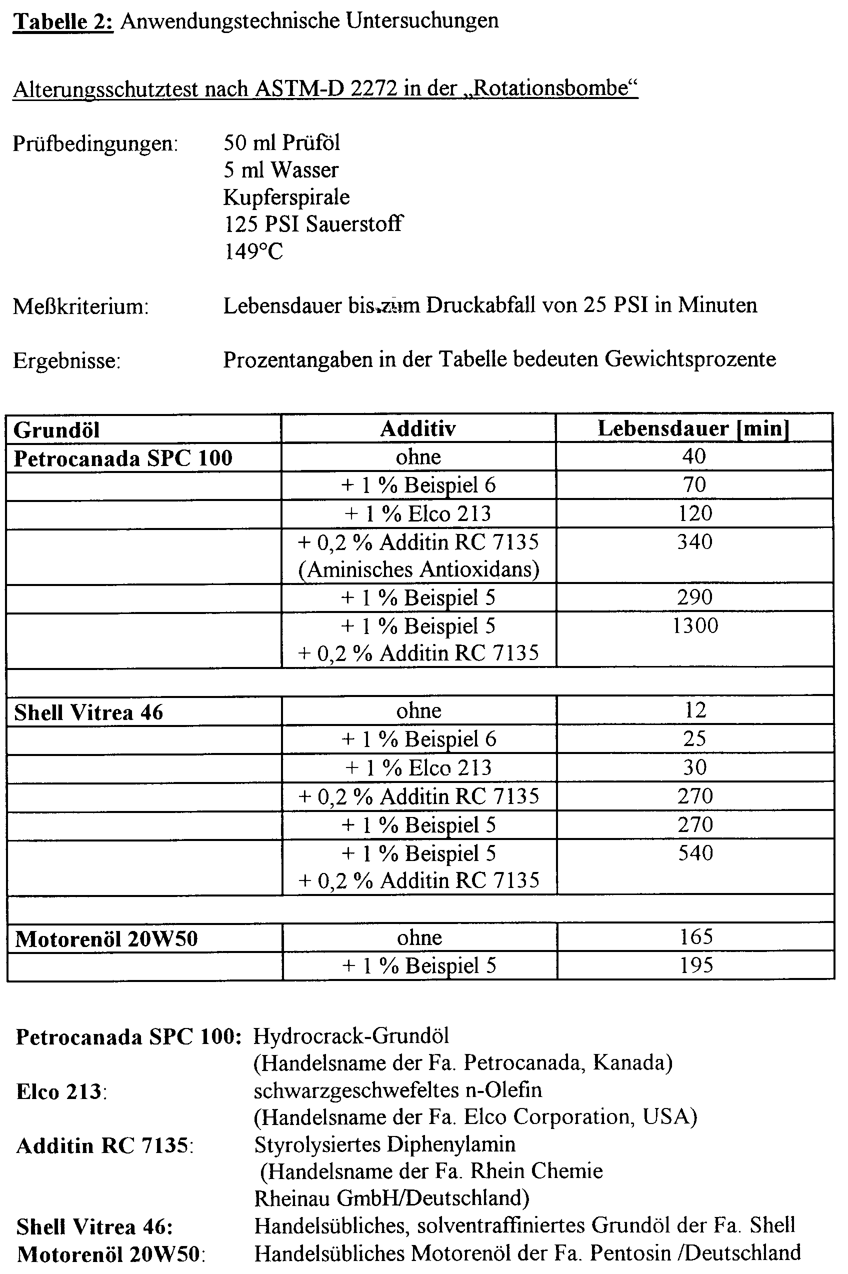

## Patentansprüche

1. Geschwefelte unverzweigte Verbindungen der Formel R₂Sₙ, worin R ein unverzweigter Alkylrest mit 4 bis 22 Kohlenstoffatomen ist und n 1,5 bis 3,5 bedeutet.

2. Verfahren zur Herstellung der geschwefelten unverzweigten Verbindungen gemäß Anspruch 1, dadurch gekennzeichnet, daß endständige, unverzweigte Olefine mit 4 bis 22 Kohlenstoffatomen, 0,5 bis 1,5 mol Schwefel und 0,4 bis 0,7 mol Schwefelwasserstoff pro mol Olefin bei 50 bis 160°C unter 2 bis 20 bar Druck 3 bis 9 Stunden in Anwesenheit von 0,01 bis 0,5 Gew.-% (bezogen auf die Summe der Einsatzstoffe) eines anionischen Katalysators im Autoklaven umgesetzt werden.

3. Verwendung der Verbindungen gemäß Anspruch 1 als Additive in Mineralölen.

4. Verwendung der Verbindungen gemäß Anspruch 1 als Anitoxidantien in Schmierstoffen.

5. Verwendung der Verbindungen gemäß Anspruch 1 in Kombination mit primären Antioxidantien als Alterungs-/Oxidationsschutz in Schmierstoffen.
